# EUROPEAN PATENT SPECIFICATION

(11) **EP 3 236 938 B1**
(45) Date of publication and mention of the grant of the patent: **27.03.2019**
(21) Application number: 15822952.6
(22) Date of filing: 24.12.2015
(51) Int. Cl.: A61K 9/00

(54) **ORAL TOPICAL AQUEOUS PHARMACEUTICAL COMPOSITIONS OF FLURBIPROFEN AND DEXPANTHENOL**
ORALE TOPISCHE WÄSSRIGE PHARMAZEUTISCHE ZUSAMMENSETZUNGEN AUS FLURBIPROFEN UND DEXPANTHENOL
COMPOSITIONS PHARMACEUTIQUES AQUEUSES TOPIQUES ORALES DE FLURBIPROFÈNE ET DE DEXPANTHÉNOL

(30) Priority: 25.12.2014 TR 201415759
(43) Date of publication of application: 01.11.2017
(73) Proprietor: Sanovel Ilac Sanayi ve Ticaret A.S., 34460 Istanbul (TR)
(72) Inventor: TÜRKYILMAZ, Ali, 34460 Istanbul (TR); PEHLIVAN AKALIN, Nur, 34460 Istanbul (TR); SUCUOGLU, Esra, 34460 Istanbul (TR); KAPLAN, Gülcan, 34460 Istanbul (TR)
(74) Representative: Sevinç, Erkan
(86) International application number: PCT/EP2015/081246
(87) International publication number: WO 2016/102708

(56) References cited:
- EP-A1- 1 974 751
- WO-A1-2005/058276

## Description

### Field of the Invention

The present invention relates to stable oral topical aqueous pharmaceutical compositions comprising flurbiprofen or pharmaceutically acceptable salts thereof and dexpanthenol or pharmaceutically acceptable salts thereof. Furthermore the present invention also relates to stable oral topical aqueous pharmaceutical compositions comprising flurbiprofen or pharmaceutically acceptable salts thereof and dexpanthenol or pharmaceutically acceptable salts thereof and chlorhexidine or pharmaceutically acceptable salts thereof. More particularly, the present invention relates to stable oral topical aqueous pharmaceutical compositions of these combinations having desired levels of solubility, enhanced taste and absorption from mucosal surface, wherein the pH of the solution is between 6 and 7.

### Background of the Invention

Flurbiprofen is a well-known non-steroidal anti-inflammatory drug which has analgesic and anti-inflammatory activities. It is used in muscle-skeletal and joint disorders such as ankylosing spondylitis, osteoarthritis and rheumatoid arthritis, in soft-tissue disorders such as sprains and strains and for postoperative pains. Its chemical structure is illustrated with Formula I given below.

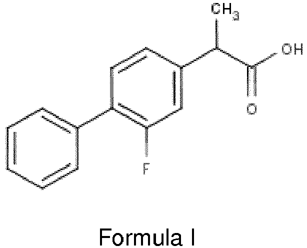

Flurbiprofen is mostly administrated orally. One disadvantage of the oral administration of flurbiprofen comprising compositions is that the patient is likely to experience unpleasant side effects, including gastrointestinal irritation. The use of flurbiprofen in treating local pains and inflammations may cause a problem especially for those who have gastrointestinal system disorders. It is possible to develop various locally-administrable topical forms of flurbiprofen, in order to avoid the systemic side-effects thereof.

Another disadvantage of flurbiprofen is that it is practically insoluble in water. This makes it difficult to prepare its aqueous solutions.

Dexpanthenol (D-panthenol) is a stable alcoholic precursor of the pantothenic acid, which represents the biologically active substance. In the tissues, including the skin, dexpanthenol is rapid and completely metabolized when applied topically. Dexpanthenol is absorbed by the skin where it is converted into Pantothenic Acid (Vitamin B5) and is thus a provitamin of B5. Dexpanthenol acts as a humectant, improving stratum corneum hydration, reducing transdermal loss of water and promoting skin elasticity. On the other hand, its cutaneous regeneration activity results from the activation of fibroblasts proliferation, which is relevant in the healing of wounds and of the injured mucous membrane.

Dexpanthenol and its salts have the disadvantage of having a bitter taste. Thus there is a need to mask the bitter taste of these compounds so that patient compliance will be increased effectively. Its chemical structure is illustrated with Formula II given below.

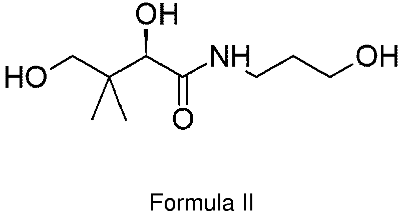

Furthermore, this formulation may also comprise chlorhexidine in addition to flurbiprofen and dexpanthenol. Chlorhexidine is an antiseptic and disinfectant against a wide variety of bacteria, fungi and some viruses. It is used clinically in various preparations for disinfecting purposes such as oral rinses and skin cleansers. It is often used in mouthwash designed for reducing dental plaque and oral bacteria and to treat and prevent gingivitis.

Chlorhexidine and its derivatives have the disadvantage that they stain the teeth with repeated use and have a bitter taste. Thus there is a need to increase the water solubility of chlorhexidine and its derivatives and to mask the bitter taste of these compounds so that they can be more incorporated into the aqueous based compositions to decrease the level of staining the teeth significantly and find greater acceptability to the user.

Accordingly, for the treatment of oral mucosa and oral cavity inflammations, stomatitis, aphtha, periodontal diseases and non-specific epidermal and muco-epidermal infections and inflammations there arises the need of an oral topical aqueous compositions comprising flurbiprofen and dexpanthenol or flurbiprofen and dexpanthenol and chlorhexidine which optimizes the delivery of them through the oral mucosal surface.

### Summary of the Invention

It is therefore an object of the present invention to provide an oral topical aqueous pharmaceutical composition of flurbiprofen or pharmaceutically acceptable salts thereof and dexpanthenol or pharmaceutically acceptable salts thereof having desired levels of solubility enhanced taste and absorption from mucosal surface.

The inventors of the present invention have discovered that by using an alkalizing agent the pH of the solution is adjusted to pH 6 to 7 thus the solubility of flurbiprofen is increased and absorption through the oral mucosal surface achieved to be at the desired levels. Therefore it is another object of the invention to provide an oral topical aqueous pharmaceutical composition of flurbiprofen or pharmaceutically acceptable salts thereof and dexpanthenol or pharmaceutically acceptable salts wherein the pH of the solution is between 6 and 7.

In addition to this, the inventors of the present invention have discovered that menthol not only masks the bitter taste of flurbiprofen and dexpanthenol but also increase the solubility and improve the clearness of the solution. Thus it is another object of the invention to provide an oral topical aqueous pharmaceutical composition of this combination comprising menthol and wherein the pH of the solution is between 6 and 7.

The invention further provides a method for the treatment of oral mucosa and oral cavity inflammations, stomatitis, aphtha, periodontal diseases and non-specific epidermal and muco-epidermal infections and inflammation using such preparations. Another object of the present invention is to provide easily applicable oral topical aqueous pharmaceutical compositions of flurbiprofen or pharmaceutically acceptable salts thereof and dexpanthenol or pharmaceutically acceptable salts thereof having desired levels of solubility and having improved taste.

Furthermore this pharmaceutical composition may also comprise chlorhexidine or pharmaceutically acceptable salts thereof in addition to flurbiprofen or pharmaceutically acceptable salts thereof and dexpanthenol or pharmaceutically acceptable salts thereof. Thus, another object of the invention is to provide a composition preventing the staining of the teeth caused by the regular use of chlorhexidine.

A further object of the present invention is to obtain a stable topical aqueous pharmaceutical composition of flurbiprofen and dexpanthenol or flurbiprofen and dexpanthenol and chlorhexidine during the shelf-life and to exhibit high safety when applied to the oral mucosal surface.

A further object of the present invention is to obtain a formulation with local anesthetic effect, with the menthol used in said combinations stimulating the receptors by which the cold sensation is perceived.

Further advantages and embodiments of the present invention will become apparent from the following description

### Detailed Description of Invention

According to the present invention, a novel oral topical aqueous pharmaceutical composition with anti-inflammatory and antiseptic activities is obtained surprisingly, which is having desired levels of solubility, enhanced taste and rapidly absorbed from the oral mucosa.

Flurbiprofen useful in accordance with this invention includes the pharmaceutically acceptable salts and esters of flurbiprofen, and further includes the conventionally used racemic mixture which comprises the S- and R- enantiomers of flurbiprofen. The topical aqueous pharmaceutical compositions of the invention comprise from 0.01 to 5.0% flurbiprofen, preferably from 0.05 to 3.0%, more preferably from 0.10 to 2.0% by weight of the total composition.

The topical aqueous pharmaceutical compositions of the invention comprise from 0.10 to 10.0% dexpanthenol or salts and esters thereof, preferably from 0.50 to 8.0%, more preferably from 1.0 to 7.5% by weight of the total composition.

The topical aqueous pharmaceutical compositions of the invention comprise an alkalizing agent to adjust the pH of the solution to pH 6-7. Adjusting the pH of the solution to about 6 to 7 pH helps to enhance the dissolution, extent of penetration into oral mucosa, especially muco-epidermal and epidermal tissue and bioavailability. It also helps to increase the solubility of flurbiprofen which is otherwise insoluble in water thus making the aqueous formulations of it difficult and may cause a blur solution. Adjusting the pH to about 6 to 7 both helps to improve the solubility of flurbiprofen, dexpanthenol and furthermore chlorhexidine and increase the rate of absorption of them from the oral mucosal surface and thus a homogenous and clear solution is also obtained.

According to this embodiment, the oral aqueous pharmaceutical composition of the present invention comprises, on a weight basis,
(i) 0.01 to 5.0% flurbiprofen or pharmaceutically acceptable salts thereof;
(ii) 0.10 to 10.0% dexpanthenol or pharmaceutically acceptable salts thereof;
(iii) 0.1 to 5.0% sodium hydroxide to adjust the pH of the solution to between 6 and 7.

Suitable alkalizing agents comprise but not limited to sodium hydroxide, calcium hydroxide, diethanolamine, monoethanolamine, potassium bicarbonate, potassium citrate, potassium hydroxide, sodium bicarbonate, sodium borate, sodium carbonate, sodium citrate dehydrate, triethanolamine and mixtures thereof. Preferably alkalizing agent is sodium hydroxide. Suitable alkalizing agents are preferably from 0.1 to 5.0%, more preferably from 1.0 to 4.0% by weight of the total composition.

In another embodiment, the topical aqueous pharmaceutical compositions of the invention comprise menthol, from 0.01 to 2.0%, preferably from 0.05 to 1.0%, more preferably from 0.1 to 0.5% by weight of the total composition. Menthol helps the composition of the present invention to improve and enhance the penetrating and spreading properties through the oral mucosal surface. It also helps to increase the solubility of the flurbiprofen and dexpanthenol and thus a homogenous and clear solution is obtained. Accordingly, menthol is used as a flavoring agent and masks the bitter taste of dexpanthenol and flurbiprofen. When other flavoring agents are used an unclear and blurry solution is obtained. The present inventors discovered that using menthol as a taste enhancer solves this problem and a clear solution is obtained as desired.

Furthermore menthol used in the formulation gives anesthetic effect at the side of administration as a result of stimulating the receptors by which cold sensation is perceived. Menthol when used with flurbiprofen and dexpanthenol, provides significant relief of pain associated with oral mucosa and oral cavity inflammations, periodontal disease and non-specific epidermal and muco-epidermal infections. Also menthol helps to mask the bad odour of the other excipients to obtain a good patient compliance when applying inside the mouth.

According to these embodiments, the oral aqueous pharmaceutical composition of the present invention comprises, on a weight basis,
(i) 0.01 to 5.0% flurbiprofen or pharmaceutically acceptable salts thereof;
(ii) 0.10 to 10.0% dexpanthenol or pharmaceutically acceptable salts thereof;
(iii) 0.01 to 2.0% menthol

In a preffered embodiment, the oral aqueous pharmaceutical composition of the present invention comprises, on a weight basis,
(i) 0.01 to 5.0% flurbiprofen or pharmaceutically acceptable salts thereof;
(ii) 0.10 to 10.0% dexpanthenol or pharmaceutically acceptable salts thereof;
(iii) 0.01 to 2.0% menthol
(iv) 0.1 to 5.0% sodium hydroxide to adjust the pH of the solution to between 6 and 7.

The pharmaceutical compositions according to the present invention may also comprise one or more pharmaceutically acceptable excipients. Such suitable pharmaceutically acceptable excipients comprise, but are not limited to surfactants, viscosity enhancers, flavoring agents, sweetening agents, solvents, and their mixtures. The pharmaceutical compositions according to the present invention further comprise a dye optionally.

Suitable solvents may comprise but not limited to ethyl alcohol, glycerin, sorbitol, propylene glycol, polyethylene glycol, isopropyl alcohol, purified water and mixtures thereof. Preferably ethyl alcohol, sorbitol, glycerin and purified water are used. Ethyl alcohol is also utilized as a microbiological preservative. Suitable solvents are used in an amount of from 5 to 45%, preferably from 10 to 35% by weight of the total composition.

Suitable preservatives, may include but not limited to methyl paraben, propyl paraben and salts thereof (e.g. sodium or potassium salts), sodium benzoate, citric acid, benzoic acid, butylated hydroxytoluene and butylated hydroxyanisole or mixtures thereof. Preferably the preservatives are methyl paraben and propyl paraben in an amount of 0.01 to 0.50 % by weight of the total composition, more preferably in an amount of 0.01 to 0.15 % by weight of the total composition.

Suitable surface active agents may comprise but not limited to polyoxyethylene castor oil derivatives, polysorbate, polyexyethylene stearates, polyoxylglycerides, glyceryl monooleate, sorbic acid, butylparaben, phospholipids and mixtures thereof. Preferably the surface active agent is polyoxyethylene castor oil derivatives, more preferably polyoxyl 40 hydrogenated castor oil. The amount of polyoxyethylene castor oil derivative is from 0.1 to 5.0%, preferably from 0.5 to 2.5% by weight of the total composition. These amounts of polyoxyethylene castor oil derivatives improve the spreading properties and minimize drying of the aqueous compositions of the present invention when it is applied inside of the mouth via spray, mouth rinse or mouthwash.

Suitable viscosity enhancers may comprise but not limited to glycerin, sodium carboxyl methyl cellulose, dextran, cellulose and derivatives, chitosan, carbomer and mixtures thereof. Preferably the viscosity enhancer is glycerin and the amount is from 5.0 to 25.0%, preferably from 10.0 to 25%, more preferably from 10.0 to 20.0% by weight of the total composition. Glycerin has also a stabilization effect when it is used in these amounts and helps the formulation to be stable over the shelf life.

Suitable flavoring agents may comprise but not limited to menthol, mint oil, eucalyptus oil, carnation oil, ginger oil, lavender oil, sweet orange oil and their mixtures. Preferably it is menthol and the amount is from 0.01 to 2.0%, preferably from 0.05 to 1.0%, more preferably from 0.1 to 0.5% by weight of the total composition.

Suitable sweetening agents may comprise but not limited to sorbitol, saccharin, saccharin sodium, aspartame, fructose, isomalt, maltitol, maltose, mannitol, sucrose, xylitol, glycerin and their mixtures, and the amount is from 0.01 to 2.0%, preferably 0.1 to 1.0% by weight of the total composition. Preferably the sweetening agent is saccharin sodium.

Suitable dyes may comprise but not limited to Patent Blue, quinoline yellow, orange G and mixtures thereof. Preferred dye is patent blue E 131.

In one embodiment, the pharmaceutical composition according to the present invention comprises most preferably on a weight basis:
(i) 0.01 to 5.0% flurbiprofen or pharmaceutically acceptable salts thereof,
(ii) 0.10 to 10.0% dexpanthenol or salts thereof,
(iii) 0.01 to 2.0% menthol,
(iv) 0.1 to 5.0% sodium hydroxide,
(v) 5.0 to 20.0 % sorbitol,
(vi) 0.01 to 2.0% saccharine sodium,
(vii) 5.0 to 25% glycerin,
(viii) 0.1 to 5.0% polyoxyl 40 hydrogenated castor oil,
(ix) 0.01 to 0.50% methyl paraben,
(x) 0.01 to 0.20% propyl paraben,
(xi) 5.0 to 15.0 % ethyl alcohol,
(xii) 0.0001 to 0.001 % patent blue

In another embodiment, this pharmaceutical composition may also comprise chlorhexidine or pharmaceutically acceptable salts thereof in addition to flurbiprofen or pharmaceutically acceptable salts thereof and dexpanthenol or pharmaceutically acceptable salts thereof which does not cause the staining of the teeth which is an unwanted effect of chlorhexidine in oral aqueous preparations.

In one embodimet, the topical aqueous pharmaceutical compositions of the invention comprise from 0.01 to 3.00% chlorhexidine or salts and esters thereof, preferably from 0.05 to 2.50%, more preferably from 0.10 to 2.0% by weight of the total composition.

According to this embodiment, the pharmaceutical composition according to the present invention comprises most preferably on a weight basis:
(i) 0.01 to 5.0% flurbiprofen or pharmaceutically acceptable salts thereof;
(ii) 0.10 to 10.0% dexpanthenol or pharmaceutically acceptable salts thereof;
(iii) 0.01 to 3.0% chlorhexidine or pharmaceutically acceptable salts thereof;

According to the more preferred embodiment, the pharmaceutical composition according to the present invention comprises most preferably on a weight basis:
(i) 0.01 to 5.00% flurbiprofen or pharmaceutically acceptable salts thereof,
(ii) 0.10 to 10.0% dexpanthenol or pharmaceutically acceptable salts thereof,
(iii) 0.01 to 3.00% chlorhexidine or pharmaceutically acceptable salts thereof,
(iv) 0.01 to 2.00% menthol,
(v) 0.1 to 5.0% sodium hydroxide,
(vi) 5.0 to 20.0 % sorbitol,
(vii) 0.01 to 2.0% saccharine sodium,
(viii) 5.0 to 25% glycerin,
(ix) 0.1 to 5.0% polyoxyl 40 hydrogenated castor oil,
(x) 5.0 to 15.0 % ethyl alcohol,
(xi) 0.0001 to 0.001% patent blue

In one embodiment, the topical compositions of the invention may be in the form of mouthwash, mouth rinse and spray.

Accordingly, the said oral topical aqueous pharmaceutical compositions of the present invention may be used for treating oral mucosa and oral cavity inflammations, stomatitis, aphtha, periodontal diseases and non-specific epidermal and muco-epidermal infections and inflammation.

This invention is further defined by reference to the following examples. Although the examples are not intended to limit the scope of the present invention, they should be considered in the light of the description detailed above.

### Example 1

### (0.25 % Flurbiprofen and 5.0% Dexpanthenol oral topical aqueous solution / 30 ml)

| **Ingredients** | **Amount (mg/ml)** |
|---|---|
| Flurbiprofen | 2.5 |
| Dexpanthenol | 50.0 |
| Sorbitol 70 | 100.0 |
| Saccharine sodium | 1.5 |
| Glycerin | 150.0 |
| Polyoxyl 40 hydrogenated castor oil | 10.0 |
| Methyl paraben | 0.9 |
| Propyl paraben | 0.2 |
| Ethyl alcohol | 80.0 |
| Patent blue E131 | 0.004 |
| Menthol | 1.6 |
| Sodium hydroxide (pH=6.7) | 30.0 - 40.0 |
| Purified water | q.s |

| | |
|---|---|
| q.s: ***Quantum*** Sufficiat (***sufficient*** quantity) | |

### Preparation of the oral topical aqueous solution:

Step 1: Flurbiprofen was dissolved in ethyl alcohol, methyl paraben and propyl paraben and stirred until it dissolves (apprx. 10 min.)

Step 2: Dexpanthenol is stirred in purified water until it dissolves (apprx. 10 min.)

Step 3: Step 1 and step 2 is mixed for 10 min.

Step 4: Adding sodium hydroxide to the solution in step 3 and stirred for 10 min.

Step 5: Polyoxyl 40 hydrogenated castor oil, glycerin and sorbitol 70 are added to the solution in step 4 and stirred for 15 min.

Step 6: Adding saccharine sodium to the solution in step 5 and stirred for 10 min. and then patent blue E131 is added and stirred further 5 more min.

Step 7: menthol is dissolved in ethyl alcohol and added to the solution in step 6 and stirred for 10 min.

Step 8: The pH of the solution was adjusted to 6.7 by adding sodium hydroxide to the solution and sufficient amount of purified water is added to the final solution to make the volume of the solution.

### Example 2

### (0.25 % Flurbiprofen, 5.0% Dexpanthenol and 0.12% Chlorhexidine oral topical aqueous solution / 30 ml)

| **Ingredients** | **Amount (mg/ml)** |
|---|---|
| Flurbiprofen | 2.5 |
| Dexpanthenol | 50.0 |
| Chlorhexidine gluconate | 1.2 |
| Sorbitol 70 | 100.0 |
| Saccharine sodium | 1.5 |
| Glycerin | 150.0 |
| Polyoxyl 40 hydrogenated castor oil | 10.0 |
| Ethyl alcohol | 80.0 |
| Patent blue E131 | 0.004 |
| Menthol | 1.6 |
| Sodium hydroxide (pH=6.7) | 30.0 - 40.0 |
| Purified water | q.s |

| | |
|---|---|
| q.s: ***Quantum*** Sufficiat (***sufficient*** quantity) | |

### Preparation of the oral topical aqueous solution:

Step 1: Flurbiprofen was dissolved in ethyl alcohol and stirred until it dissolves (apprx. 10 min.)

Step 2: Adding sodium hydroxide to the solution of step 1 and stirred for 10 min.

Step 3: Polyoxyl 40 hydrogenated castor oil, glycerin, sorbitol 70 and purified water are added to the solution in step 2 and stirred for 15 min.

Step 4: Adding chlorhexidine gluconate with purified water to the solution in step 3 and stirred for 10 min. **(Mixture 1)**

Step 5: Dexpanthenol is dissolved in purified water in another container **(Mixture 2)**

Step 6: Mixture 1 and mixture 2 is mixed for 10 min.

Step 7: Adding saccharine sodium to the solution in step 6 and stirred for 10 min. and then patent blue E131 is added and stirred further 5 more min.

Step 8: Menthol is dissolved in ethyl alcohol and added to the solution in step 7 and stirred for 10 min.

Step 9: The pH of the solution was adjusted to 6.7 by adding sodium hydroxide to the solution and sufficient amount of purified water is added to the final solution to make the volume of the solution.

## Claims

1. An oral topical aqueous pharmaceutical composition comprising:
(i) flurbiprofen or pharmaceutically acceptable salts thereof;
(ii) dexpanthenol or pharmaceutically acceptable salts thereof.

2. The composition according to claim 1, wherein the pH of the solution is between 6 and 7.

3. The composition according to claim 1 or 2 comprising on a weight basis,
(i) 0.01 to 5.0% flurbiprofen or pharmaceutically acceptable salts thereof;
(ii) 0.10 to 10.0% dexpanthenol or pharmaceutically acceptable salts thereof;
(iii) 0.1 to 5.0% sodium hydroxide to adjust the pH of the solution to between 6 and 7.

4. The composition according to claim 1, further comprising menthol.

5. The composition according to claim 4, comprising on a weight basis,
(i) 0.01 to 5.0% flurbiprofen or pharmaceutically acceptable salts thereof;
(ii) 0.10 to 10.0% dexpanthenol or pharmaceutically acceptable salts thereof;
(iii) 0.01 to 2.0% menthol

6. The composition according to claim 1 to 5 comprising on a weight basis,
(i) 0.01 to 5.0% flurbiprofen or pharmaceutically acceptable salts thereof;
(ii) 0.10 to 10.0% dexpanthenol or pharmaceutically acceptable salts thereof;
(iii) 0.01 to 2.0% menthol
(iv) 0.1 to 5.0% sodium hydroxide to adjust the pH of the solution to between 6 and 7.

7. The composition according to claims 1 to 6, further comprising at least one solvent in an amount of from 5.0 to 45.0 % by weight of the total composition.

8. The composition according to claim 7, wherein the solvent is selected from a group comprising ethyl alcohol, glycerin, sorbitol, polyethylene glycol, propylene glycol, isopropyl alcohol, purified water and mixtures thereof, preferably
the solvent is ethyl alcohol, glycerin and sorbitol.

9. The composition according to claims 1 to 8, further comprising at least one sweetening agent in an amount of from 0.01 to 2.0% by weight of the total composition.

10. The composition according to claim 9, wherein the sweetening agent is selected from the group comprising saccharine sodium, sorbitol, aspartame, fructose, isomalt, maltitol, maltose, mannitol, sucrose, xylitol, glycerin and their mixtures; preferably the sweetening agent is saccharine sodium.

11. The composition according to claims 1 to 10, further comprising at least one surface active agent in an amount of from 0.1 to 5.0% by weight of the total composition.

12. The composition according to claim 11, wherein the surface active agent is selected from the group comprising, polyoxyethylene castor oil derivatives, polysorbate, polyoxyethylene stearates, polyoxylglycerides, glyceryl monooleate, sorbic acid, butylparaben, phospholipids and mixtures thereof; preferably the surface active agent is polyoxyethylene castor oil derivatives; more preferably polyoxyl 40 hydrogenated castor oil.

13. The composition according to claims 1 to 12, further comprising at least one preservative in an amount of from 0.01 to 0.50 % by weight of the total composition.

14. The composition according to claim 13, wherein the preservative is selected from the group comprising methyl paraben, propyl paraben and salts thereof, sodium benzoate, citric acid, benzoic acid, butylated hydroxytoluene and butylated hydroxyanisole or mixtures thereof; preferably the preservatives are methyl paraben and propyl paraben.

15. The composition according to any of the preceding claims, comprising on a weight basis:
(i) 0.01 to 5.0% flurbiprofen or pharmaceutically acceptable salts thereof;
(ii) 0.10 to 10.0% dexpanthenol or pharmaceutically acceptable salts thereof;
(iii) 0.01 to 2.0% menthol
(iv) 0.1 to 5.0% sodium hydroxide
(v) 5.0 to 20.0 % sorbitol;
(vi) 0.01 to 2.0% saccharine sodium;
(vii) 5.0 to 25.0% glycerin;
(viii) 0.1 to 5.0% polyoxyl 40 hydrogenated castor oil;
(ix) 0.01 to 0.50% methyl paraben,
(x) 0.01 to 0.20% propyl paraben,
(xi) 5.0 to 15.0 % ethyl alcohol
(xii) 0.0001 to 0.001% patent blue

16. The composition according to claim 1, further comprising chlorhexidine or pharmaceutically acceptable salts thereof.

17. The composition according to claim 16 comprising on a weight basis,
(i) 0.01 to 5.0% flurbiprofen or pharmaceutically acceptable salts thereof;
(ii) 0.10 to 10.0% dexpanthenol or pharmaceutically acceptable salts thereof;
(iii) 0.01 to 3.0% chlorhexidine or pharmaceutically acceptable salts thereof;

18. The composition according to claim 17, comprising on a weight basis:
(i) 0.01 to 5.0% flurbiprofen or pharmaceutically acceptable salts thereof;
(ii) 0.10 to 10.0% dexpanthenol or pharmaceutically acceptable salts thereof;
(iii) 0.01 to 3.0% chlorhexidine or pharmaceutically acceptable salts thereof;
(iv) 0.01 to 2.0% menthol
(v) 0.1 to 5.0% sodium hydroxide
(vi) 5.0 to 20.0 % sorbitol;
(vii) 0.01 to 2.0% saccharine sodium;
(viii) 5.0 to 25.0% glycerin;
(ix) 0.1 to 5.0% polyoxyl 40 hydrogenated castor oil;
(x) 5.0 to 15.0 % ethyl alcohol
(xi) 0.0001 to 0.001% patent blue

19. The composition according to any one of the preceding claims, wherein said composition is in the form of a mouthwash, mouth rinse or spray.

20. The composition according to any one of the preceding claims, for use in the treatment of oral mucosa and oral cavity inflammations, stomatitis, aphtha, periodontal diseases and non-specific epidermal and muco-epidermal infections and inflammation.

## Patentansprüche

1. Orale topische wässrige pharmazeutische Zusammensetzung, umfassend:
(i) Flurbiprofen oder pharmazeutisch verträgliche Salze davon;
(ii) Dexpanthenol oder pharmazeutisch verträgliche Salze davon.

2. Zusammensetzung nach Anspruch 1, wobei der pH-Wert der Lösung zwischen 6 und 7 liegt.

3. Zusammensetzung nach Anspruch 1 oder 2, umfassend auf Gewichtsbasis,
(i) 0,01 bis 5,0 % Flurbiprofen oder pharmazeutisch verträgliche Salze davon;
(ii) 0,10 bis 10,0 % Dexpanthenol oder pharmazeutisch verträgliche Salze davon;
(iii) 0,1 bis 5,0 % Natriumhydroxid, um den pH-Wert der Lösung auf zwischen 6 und 7 einzustellen.

4. Zusammensetzung nach Anspruch 1, ferner umfassend Menthol.

5. Zusammensetzung nach Anspruch 4, umfassend auf Gewichtsbasis,
(i) 0,01 bis 5,0 % Flurbiprofen oder pharmazeutisch verträgliche Salze davon;
(ii) 0,10 bis 10,0 % Dexpanthenol oder pharmazeutisch verträgliche Salze davon;
(iii) 0,01 bis 2,0 % Menthol

6. Zusammensetzung nach Anspruch 1 bis 5, umfassend auf Gewichtsbasis,
(i) 0,01 bis 5,0 % Flurbiprofen oder pharmazeutisch verträgliche Salze davon;
(ii) 0,10 bis 10,0 % Dexpanthenol oder pharmazeutisch verträgliche Salze davon;
(iii) 0,01 bis 2,0 % Menthol
(iv) 0,1 bis 5,0 % Natriumhydroxid, um den pH-Wert der Lösung auf zwischen 6 und 7 einzustellen.

7. Zusammensetzung nach den Ansprüchen 1 bis 6, ferner umfassend mindestens ein Lösungsmittel in einer Menge von 5,0 bis 45,0 Gew.-% der Gesamtzusammensetzung.

8. Zusammensetzung nach Anspruch 7, wobei das Lösungsmittel ausgewählt ist aus einer Gruppe, die Ethylalkohol, Glycerin, Sorbit, Polyethylenglykol, Propylenglykol, Isopropylalkohol, gereinigtes Wasser und Mischungen derselben umfasst, vorzugsweise das Lösungsmittel Ethylalkohol, Glycerin und Sorbitol ist.

9. Zusammensetzung nach den Ansprüchen 1 bis 8, ferner umfassend mindestens ein Süßungsmittel in einer Menge von 0,01 bis 2,0 Gew.-% der Gesamtzusammensetzung.

10. Zusammensetzung nach Anspruch 9, wobei das Süßungsmittel ausgewählt ist aus der Gruppe umfassend Natriumsaccharin, Sorbit, Aspartam, Fructose, Isomalt, Maltit, Maltose, Mannit, Saccharose, Xylit, Glycerin und deren Mischungen; vorzugsweise ist das Süßungsmittel Natriumsaccharin.

11. Zusammensetzung nach den Ansprüchen 1 bis 10, ferner umfassend mindestens ein oberflächenaktives Mittel in einer Menge von 0,1 bis 5,0 Gew.-% der Gesamtzusammensetzung.

12. Zusammensetzung nach Anspruch 11, wobei das oberflächenaktive Mittel ausgewählt ist aus der Gruppe, die Polyoxyethylen-Rizinusölderivate, Polysorbat, Polyoxyethylen-Stearate, Polyoxylglyceride, Glycerinmonooleat, Sorbinsäure, Butylparaben, Phospholipide und Mischungen davon umfasst; vorzugsweise ist das oberflächenaktive Mittel Polyoxyethylen-Rizinusölderivate; insbesondere Polyoxyl-40 hydriertes Rizinusöl.

13. Zusammensetzung nach den Ansprüchen 1 bis 12, ferner umfassend mindestens ein Konservierungsmittel in einer Menge von 0,01 bis 0,50 Gew.-% der Gesamtzusammensetzung.

14. Zusammensetzung nach Anspruch 13, wobei das Konservierungsmittel ausgewählt ist aus der Gruppe umfassend Methylparaben, Propylparaben und Salze davon, Natriumbenzoat, Zitronensäure, Benzoesäure, butyliertes Hydroxytoluol und butyliertes Hydroxyanisol oder Mischungen davon; vorzugsweise sind die Konservierungsmittel Methylparaben und Propylparaben.

15. Zusammensetzung nach einem der vorhergehenden Ansprüche, umfassend auf Gewichtsbasis:
(i) 0,01 bis 5,0 % Flurbiprofen oder pharmazeutisch verträgliche Salze davon;
(ii) 0,10 bis 10,0 % Dexpanthenol oder pharmazeutisch verträgliche Salze davon;
(iii) 0,01 bis 2,0 % Menthol
(iv) 0,1 bis 5,0 % Natriumhydroxid
(v) 5,0 bis 20,0 % Sorbitol;
(vi) 0,01 bis 2,0 % Natriumsaccharin;
(vii) 5,0 bis 25,0 % Glycerin;
(viii) 0,1 bis 5,0 % Polyoxyl-40 hydriertes Rizinusöl;
(ix) 0,01 bis 0,50 % Methylparaben,
(x) 0,01 bis 0,20 % Propylparaben,
(xi) 5,0 bis 15,0 % Ethylalkohol
(xii) 0,0001 bis 0,001 % Patentblau

16. Zusammensetzung nach Anspruch 1, ferner umfassend Chlorhexidin oder pharmazeutisch verträgliche Salze davon.

17. Zusammensetzung nach Anspruch 16, umfassend auf Gewichtsbasis,
(i) 0,01 bis 5,0 % Flurbiprofen oder pharmazeutisch verträgliche Salze davon;
(ii) 0,10 bis 10,0 % Dexpanthenol oder pharmazeutisch verträgliche Salze davon;
(iii) 0,01 bis 3,0 % Chlorhexidin oder pharmazeutisch verträgliche Salze davon;

18. Zusammensetzung nach Anspruch 17, umfassend auf Gewichtsbasis:
(i) 0,01 bis 5,0 % Flurbiprofen oder pharmazeutisch verträgliche Salze davon;
(ii) 0,10 bis 10,0 % Dexpanthenol oder pharmazeutisch verträgliche Salze davon;
(iii) 0,01 bis 3,0 % Chlorhexidin oder pharmazeutisch verträgliche Salze davon;
(iv) 0,01 bis 2,0 % Menthol
(v) 0,1 bis 5,0 % Natriumhydroxid
(vi) 5,0 bis 20,0 % Sorbitol;
(vii) 0,01 bis 2,0 % Natriumsaccharin;
(viii) 5,0 bis 25,0 % Glycerin;
(ix) 0,1 bis 5,0 % Polyoxyl-40 hydriertes Rizinusöl;
(x) 5,0 bis 15,0 % Ethylalkohol
(xi) 0,0001 bis 0,001 % Patentblau

19. Zusammensetzung nach einem der vorhergehenden Ansprüche, wobei die Zusammensetzung in Form eines Mundwassers, einer Mundspülung oder eines Sprays vorliegt.

20. Zusammensetzung nach einem der vorhergehenden Ansprüche zur Verwendung bei der Behandlung von Entzündungen der Mundschleimhaut und der Mundhöhle, Stomatitis, Aphthe, Parodontalerkrankungen und unspezifischen epidermalen und mukoepidermalen Infektionen und Entzündungen.

## Revendications

1. Composition pharmaceutique aqueuse topique orale comprenant :
(i) du flurbiprofène ou des sels pharmaceutiquement acceptables de celui-ci ;
(ii) du dexpanthénol ou des sels pharmaceutiquement acceptables de celui-ci.

2. Composition selon la revendication 1, dans laquelle le pH de la solution est entre 6 et 7.

3. Composition selon l'une des revendications 1 ou 2 comprenant, sur une base en poids,
(i) 0,01 à 5,0 % de flurbiprofène ou de sels pharmaceutiquement acceptables de celui-ci ;
(ii) 0,10 à 10,0 % de dexpanthénol ou de sels pharmaceutiquement acceptables de celui-ci ;
(iii) 0,1 à 5,0 % d'hydroxyde de sodium pour ajuster le pH de la solution à entre 6 et 7.

4. Composition selon la revendication 1, comprenant en outre du menthol.

5. Composition selon la revendication 4, comprenant, sur une base en poids,
(i) 0,01 à 5,0 % de flurbiprofène ou de sels pharmaceutiquement acceptables de celui-ci ;
(ii) 0,10 à 10,0 % de dexpanthénol ou de sels pharmaceutiquement acceptables de celui-ci ;
(iii) 0,01 à 2,0 % de menthol.

6. Composition selon l'une des revendications 1 à 5, comprenant, sur une base en poids,
(i) 0,01 à 5,0 % de flurbiprofène ou de sels pharmaceutiquement acceptables de celui-ci ;
(ii) 0,10 à 10,0 % de dexpanthénol ou de sels pharmaceutiquement acceptables de celui-ci ;
(iii) 0,01 à 2,0 % de menthol ;
(iv) 0,1 à 5,0 % d'hydroxyde de sodium pour ajuster le pH de la solution à entre 6 et 7.

7. Composition selon l'une des revendications 1 à 6, comprenant en outre au moins un solvant dans une quantité de 5,0 à 45,0 % en poids de la composition totale.

8. Composition selon la revendication 7, dans laquelle le solvant est choisi dans un groupe comprenant l'alcool éthylique, le glycérol, le sorbitol, le polyéthylène glycol, le propylène glycol, l'alcool isopropylique, l'eau purifiée et leurs mélanges, de préférence le solvant est l'alcool éthylique, le glycérol et le sorbitol.

9. Composition selon l'une des revendications 1 à 8, comprenant en outre au moins un agent édulcorant dans une quantité de 0,01 à 2,0 % en poids de la composition totale.

10. Composition selon la revendication 9, dans laquelle l'agent édulcorant est choisi dans le groupe comprenant la saccharine sodique, le sorbitol, l'aspartame, le fructose, l'isomalt, le maltitol, le maltose, le mannitol, le saccharose, le xylitol, le glycérol et leurs mélanges ; de préférence, l'agent édulcorant est la saccharine sodique.

11. Composition selon l'une des revendications 1 à 10, comprenant en outre au moins un agent tensio-actif dans une quantité de 0,1 à 5,0 % en poids de la composition totale.

12. Composition selon la revendication 11, dans laquelle l'agent tensio-actif est choisi dans le groupe comprenant les dérivés d'huile de ricin polyoxyéthylénés, le polysorbate, les stéarates polyoxyéthylénés, les polyoxylglycérides, le monooléate de glycéryle, l'acide sorbique, le butylparabène, les phospholipides et leurs mélanges, de préférence, l'agent tensio-actif consiste en dérivés d'huile de ricin polyoxyéthylénés, de manière davantage préférée, en l'huile de ricin hydrogénée de polyoxyl 40.

13. Composition selon l'une des revendications 1 à 12, comprenant en outre au moins un conservateur dans une quantité de 0,01 à 0,50 % en poids de la composition totale.

14. Composition selon la revendication 13, dans laquelle le conservateur est choisi dans le groupe comprenant le méthyl parabène, le propyl parabène et leurs sels, le benzoate de sodium, l'acide citrique, l'acide benzoïque, l'hydroxytoluène butylé et l'hydroxyanisole butylé ou leurs mélanges, de préférence, les conservateurs sont le méthyl parabène et le propyl parabène.

15. Composition selon l'une quelconque des revendications précédentes, comprenant, sur une base en poids :
(i) 0,01 à 5,0 % de flurbiprofène ou de sels pharmaceutiquement acceptables de celui-ci ;
(ii) 0,10 à 10,0 % de dexpanthénol ou de sels pharmaceutiquement acceptables de celui-ci ;
(iii) 0,01 à 2,0 % de menthol ;
(iv) 0,1 à 5,0 % d'hydroxyde de sodium ;
(v) 5,0 à 20,0 % de sorbitol ;
(vi) 0,01 à 2,0 % de saccharine sodique ;
(vii) 5,0 à 25,0 % de glycérol ;
(viii) 0,1 à 5,0 % d'huile de ricin hydrogénée de polyoxyl 40 ;
(ix) 0,01 à 0,50 % de méthyl parabène,
(x) 0,01 à 0,20 % de propyl parabène,
(xi) 5,0 à 15,0 % d'alcool éthylique ;
(xii) 0,0001 à 0,001 % de bleu patenté.

16. Composition selon la revendication 1, comprenant en outre de la chlorhexidine ou des sels pharmaceutiquement acceptables de celle-ci.

17. Composition selon la revendication 16, comprenant, sur une base en poids,
(i) 0,01 à 5,0 % de flurbiprofène ou de sels pharmaceutiquement acceptables de celui-ci ;
(ii) 0,10 à 10,0 % de dexpanthénol ou de sels pharmaceutiquement acceptables de celui-ci ;
(iii) 0,01 à 3,0 % de chlorhexidine ou de sels pharmaceutiquement acceptables de celle-ci.

18. Composition selon la revendication 17, comprenant, sur une base en poids :
(i) 0,01 à 5,0 % de flurbiprofène ou de sels pharmaceutiquement acceptables de celui-ci ;
(ii) 0,10 à 10,0 % de dexpanthénol ou de sels pharmaceutiquement acceptables de celui-ci ;
(iii) 0,01 à 3,0 % de chlorhexidine ou de sels pharmaceutiquement acceptables de celle-ci ;
(iv) 0,01 à 2,0 % de menthol ;
(v) 0,1 à 5,0 % d'hydroxyde de sodium ;
(vi) 5,0 à 20,0 % de sorbitol ;
(vii) 0,01 à 2,0 % de saccharine sodique ;
(viii) 5,0 à 25,0 % de glycérol ;
(ix) 0,1 à 5,0 % d'huile de ricin hydrogénée de polyoxyl 40 ;
(x) 5,0 à 15,0 % d'alcool éthylique ;
(xi) 0,0001 à 0,001 % de bleu patenté.

19. Composition selon l'une quelconque des revendications précédentes, ladite composition étant sous la forme d'un bain de bouche, d'un rince-bouche ou d'une pulvérisation.

20. Composition selon l'une quelconque des revendications précédentes, pour une utilisation dans le traitement d'inflammations de la muqueuse buccale et de la cavité buccale, de la stomatite, de l'aphte, de maladies parodontales et d'infections et inflammation épidermiques et muco-épidermiques non spécifiques.
